# EUROPEAN PATENT APPLICATION

(11) **EP 1 634 557 A1**
(43) Date of publication of application: **15.03.2006**
(21) Application number: 05460016.8
(22) Date of filing: 14.06.2005
(51) Int. Cl.: A61F 13/15

(54) **The way, the method of unrolling elastic material in the industry of expendable baby napkin production**

(30) Priority: 12.09.2004 PL 36980104
(71) Applicant: Borczuch, Lech Robert, 05-220 Zielonka (PL)
(72) Inventor: Borczuch, Lech Robert, 05-220 Zielonka (PL)

(57) **Abstract**

The method consists in application of the rule of unrolling elastic material (lycra elastic) from immovable, for example, conic shaped rollers in the industry of expendable baby napkin production. Immovable material with approachable endings enables inclusion of that material and bonding thereof in the course of a production line, so production process interruption is avoided and no costs related thereto are incurred. Currently, material rollers are movable and the process of bonding consists in interruption of a production process and in replacement of empty rollers with new ones on which material is rolled. PICTORIAL FIGURE. The substance of this reservation is the way, the method, so the drawing included hereafter presents only the proposed solution of feeding the material in the form of elastic used for production of expendable baby napkins. Without any detail construction specifications, dimensions or specific features being presented.

## Description

### THE DESCRIPTION OF A SOLUTION

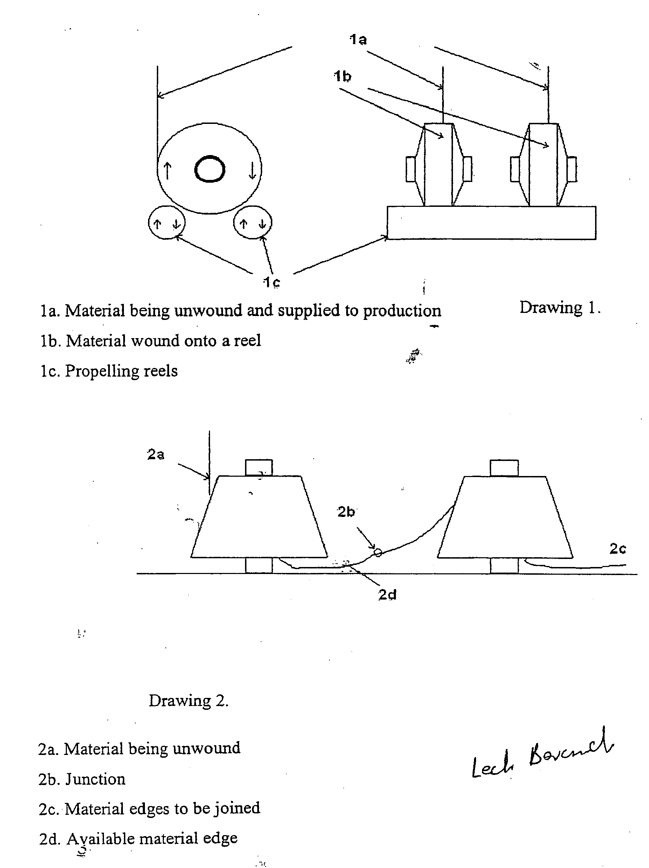

The method having been invented by me consists in application of the rule of unrolling elastic material (lycra elastic) from immovable, for example, conic shaped rollers in the industry of expendable baby napkin production. Immovable material with approachable endings enables inclusion of that material and bonding thereof in the course of a production line, so production process interruption is avoided and no costs related thereto are incurred. Until now, the method of partly unapproachable, movable rollers replaceable during a production line stoppage has been employed. Figure 1: The Pictorial Presentation of the Heretofore Method (publication is not allowed). The idea of application of immovable rollers instead of movable ones enables continuity of a production process which translates directly into remarkable reduction of costs and contributes to impressive productivity ratio increase. New conic-shaped rollers should be located in an approachable place, so binding of the ending of material being unrolled with the beginning of prepared material could be possible. Figure 2: The Pictorial Presentation of the New Method of Material Feeding for Production. The solution can be utilized by an industry and the outcome of it is reduction of production costs resulting from assurance of production continuity based on art. 27.

## Claims

1. Application No.: The way, the method of unrolling elastic material in the industry of expendable baby napkin production, that is subjected to reservation and right protection, defines the following action: *bonding (for example, by means of binding or gluing) elastic material in the progress of a production cycle and in the progress of material unrolling with the use of immovable, connectable rollers for material instead of movable ones.*
